# EUROPEAN PATENT APPLICATION

(11) **EP 4 752 225 A1**
(43) Date of publication of application: **03.06.2026**
(21) Application number: 24216159.4
(22) Date of filing: 28.11.2024
(51) Int. Cl.: C12N 15/82, A01H 5/00

(54) **NOVEL PLANT PROMOTERS**

(71) Applicant: KWS SAAT SE & Co. KGaA, 37574 Einbeck (DE)
(72) Inventor: Streitner, Corinna, 49328 Melle (DE); Weltmeier, Fridtjof, 37574 Einbeck (DE)
(74) Representative: Eisenführ Speiser

(57) **Abstract**

The present invention relates to the field of promoter activation to increase expression of a target gene in a plant. More specifically, the present invention provides a method for increasing the expression level of an endogenous polynucleotide of interest in a genomic locus of a plant, wherein the native promoter is screened for a suitable motif, which can, preferably by a single point mutation, be modified to increase the expression of the endogenous polynucleotide of interest and then the mutation is introduced, preferably by TILLING. The invention also provides a method of producing a modified promoter, a modified promoter as well as a method of screening a promoter endogenous to a plant for a motif, which can be modified by introducing one or more point mutations to create a motif, which increases the expression of the polynucleotide under the control of the promoter with respect to the unmodified promoter.

## Description

### Technical Field

The present invention relates to the field of promoter activation to increase expression of a target gene in a plant. More specifically, the present invention provides a method for increasing the expression level of an endogenous polynucleotide of interest in a genomic locus of a plant, wherein the native promoter is screened for a suitable motif, which can, preferably by a single point mutation, be modified to increase the expression of the endogenous polynucleotide of interest and then the mutation is introduced, preferably by TILLING. The invention also provides a method of producing a modified promoter, a modified promoter as well as a method of screening a promoter endogenous to a plant for a motif, which can be modified by introducing one or more point mutations to create a motif, which increases the expression of the polynucleotide under the control of the promoter with respect to the unmodified promoter.

### Background of the invention

The expression levels of many genes in an organism depend on different factors such as developmental stages or physiologic and environmental conditions. The expression of one gene can be induced under certain circumstances and completely shut down if the circumstances change. The starting point for gene expression, the transcription of a gene, is regulated by a range of different mechanisms, which usually involve the promoter region harbouring the transcription start site (TSS). While some promoters are active in all circumstances (constitutive promoters), others are tightly regulated and only respond to certain stimuli. Transcription factors bind to specific DNA sequences and activate or repress transcription (trans-acting factors). Promoter sequences therefore carry a number of binding sites for trans-acting factors, so called cis-regulatory elements.

Being able to modulate the expression of certain genes in an organism opens up a range of opportunities to improve biotechnological processes or agricultural yields. Therefore, new technologies are continuously sought, which allow to specifically control expression levels of a target gene. Promoters are an obvious target for such approaches, but up to date, there is still little known about the possibilities of activating endogenous promoters by minimal modification. No generic approach for activation of gene expression by introduction of only one or a few point mutations is currently available.

It is known that increased expression can be achieved by using strong promoters, e.g. the 35S promoter. Different translation enhancing elements have been described to be useful for expressing high levels of protein in plant cells, as parts of transgenes, or in viral expression vectors (e.g. the 5' untranslated leader of tobacco mosaic virus RNA which consists of a 68-base sequence (see Ofoghi et al., 2005. Comparison of tobacco etch virus and tobacco mosaic virus enhancers for expression of human calcitonin gene in transgenic potato plant. In Key Engineering Materials (Vol. 277, pp. 7-11). Trans Tech Publications). However, these elements are relatively large. The same is true for enhancing promoter elements like the 35S enhancer, or introns reported to increase expression, e.g. adh1 intron from Zea mays (Callis et al, 1987. Introns increase gene expression in cultured maize cells. Genes & development, 1(10), 1183-1200).

Crop traits can be improved by increased ectopic expression of a trait gene. As an example, Sun et al. (Nature comm., 2017, doi: 10.1038/ncomms14752) reported that increased expression of maize PLASTOCHRON1 enhances biomass and seed yield. They increased expression by a transgenic approach, using the GA2ox promoter. This transgenic approach to increase ectopic expression of a trait gene has the limitation that planting of transgenic plants has high regulatory requirements.

Zhang et al. found in the genus Malus, an allelic variation of the Iron-Regulated Transporter1 (IRT1) promoter in which a TATA box insertion has been identified (Plant Physiology, 2017, Vol. 173, 715-727, doi: 10.1104/pp.16.01504). Further results suggest that this insertion seems to be causative for a slight upregulation of the promoter activity (~1.5 fold). It is also possible that the increased promoter activity is caused by the increased expression of the potential TATA-box binding proteins TFIID, which activates also the IRT1 promoter.

It has since been established that certain activating elements comprising TATA box motifs, can be inserted into endogenous promoter sequences in order to increase the expression of endogenous genes (WO2019/185609A1). However, for regulatory reasons, it is desirable to introduce only minimal modifications into the genome of the target plant and not insert several nucleotide long stretches of foreign DNA. On the other hand, according to WO 2019/185609A1, in order to provide the desired effect, the activating element should comprise one or more TATA box motif(s) having a relative score of greater than 0.8 when matching or aligning the one or more TATA box motif(s) to a TATA box consensus, i.e. an ideal TATA box motif. This means that, if only a minimal modification such as a single point mutation is desired, the target promoter sequence would need to already have an "almost ideal" TATA box motif, where only one single point mutation is necessary to increase the score to at least 0.8. Of course, for most native promoter sequences, this is not the case.

In view of the increasing demand for new plants with improved properties being resistant to various kinds of biotic stress caused by pathogens feeding on the plant or plant diseases and abiotic stress, e.g. due to drought and increasing temperatures, more detailed knowledge on promoter sequences influencing the expression of genes of interest in a targeted way are urgently needed.

It was therefore an objective of the present invention to provide a novel approach for promoter activation by minimal modification, which is widely applicable, preferably to any promoter sequence. In particular, the modification required for an increase of expression should only be one or a few point mutation(s), which can be introduced by mutagenesis without using any genome editing techniques.

In the context of the present invention, it was surprisingly found that TATA box motifs, with larger deviations from an "ideal" TATA box consensus, can still provide a significant increase in expression of a gene of interest. This novel finding opens up a whole new range of possibilities to activate endogenous plant promoters by minimal modification since it should be possible to identify a suitable target motif for modification in almost any promoter sequence. Even though no "ideal" TATA box motif is used, still a significant and remarkable increase in expression can be achieved, which may well be sufficient or even preferred for a certain application and expands the toolbox of promoters suitable for modulating gene expression in a plant cell of interest.

### Summary of the invention

The above objects have been solved, in one aspect, by providing a method for increasing the expression level of an endogenous polynucleotide of interest in a genomic locus of a plant, comprising the steps:
(i) identifying in the promoter region of the genomic locus, a range of nucleotide positions, in which the introduction of a TATA-box motif of the sequence YTATAWAWA leads to an increased expression of the polynucleotide of interest compared to the unmodified genomic locus;
(ii) screening the range of nucleotide positions identified in step i) to identify a nine nucleotide motif, which can be modified by introducing one or more point mutations to create a nine nucleotide motif having at least one nucleotide and at most three nucleotides, which deviate(s) in positions 2 to 7 from a YTATAWAWA motif; and
(iii) introducing, simultaneously and/or subsequently, one or more point mutations, to create a nine nucleotide motif having at least one nucleotide and at most three nucleotides, which deviate(s) in positions 2 to 7 from the YTATAWAWA motif within the range of nucleotide positions identified in step i);
(iv) optionally: obtaining a modified promoter region increasing the expression level of an endogenous polynucleotide of interest in a genomic locus of a plant.

In one embodiment of the method described above, three point mutations, preferably two point mutations, more preferably only one point mutation is/are introduced in step (iii).

In another embodiment of the method according to any of the embodiments described above, the point mutation or at least one or at least two point mutation(s) or all point mutations is/are a C to T exchange, preferably introduced by TILLING.

In a further embodiment of the method according to any of the embodiments described above, the nine nucleotide motif having at least one nucleotide and at most three nucleotides, which deviate(s) in positions 2 to 7 from the YTATAWAWA motif has a G in position 7.

In one embodiment of the method according to any of the embodiments described above, the range of nucleotide positions identified in step (i) is between minus 400 nucleotides and minus 50 nucleotides upstream of the start codon of the gene product encoded by the polynucleotide of interest.

In another embodiment of the method according to any of the embodiments described above, the nine nucleotide motif having at least one nucleotide and at most three nucleotides, which deviate(s) in positions 2 to 7 from the YTATAWAWA motif is selected from the group consisting of CTATAAGTA, CTATATGTA, CTATAAGAA, CTATATGAA, CCATAAGTA, CTTTAAGTA, CTACAAGTA, CTATTAGTA, CTTTATGTA, CTATTTGTA, CTTTAAGAA, CATTAAGTA, CCTTAAGTA, CGTTAAGTA, CTTCAAGTA, CCTTAAATA, CTTTAAATA, CTACTTATA and CTATTTATA.

In a further embodiment of the method according to any of the embodiments described above, the plant is selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus, Beta spp., including Beta vulgaris,* Beta vulgaris subsp. vulgaris *(e.g. Swiss chard, red beet), Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Spinacia oleracea, plants of the family Cucurbitaceae (e,g, Cucumis sativus, Cucumis melo, Citrullus lanatus), plants of the genus Capsicum (e.g. Capsicum annuum), plants of the genus Phaseolus (e.g. P. vulgaris) Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* preferably *Beta vulgaris* and *Zea mays.*

In yet another embodiment of the method according to any of the embodiments described above, the polynucleotide of interest is selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance or tolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content.

In another aspect, the present invention relates to a method of producing a modified promoter region comprising the steps:
(i) identifying in a promoter endogenous to a plant, a range of nucleotide positions, in which the introduction of a TATA-box motif of the sequence YTATAWAWA leads to an increased expression of the polynucleotide under the control of the promoter;
(ii) screening the range of nucleotide positions identified in step i) to identify a nine nucleotide motif, which can be modified by introducing one or more point mutations to create a nine nucleotide motif having at least one nucleotide and at most three nucleotides, which deviate(s) in positions 2 to 7 from a YTATAWAWA motif; and
(iii) introducing, simultaneously and/or subsequently, one or more point mutations, to create a nine nucleotide motif having at least one nucleotide and at most three nucleotides, which deviate(s) in positions 2 to 7 from the YTATAWAWA motif within the range of nucleotide positions identified in step i);
(iv) optionally: obtaining a modified promoter region increasing the expression level of an endogenous polynucleotide of interest in a genomic locus of a plant.

In one embodiment of the method described above, three point mutations, preferably two point mutations, more preferably only one point mutation is/are introduced in step (iii).

In another embodiment of the method described above, the point mutation or at least one or at least two point mutation(s) or all point mutations is/are a C to T exchange, preferably introduced by TILLING.

In yet another embodiment of the method described above, the nine nucleotide motif having at least one nucleotide and at most three nucleotides, which deviate(s) in positions 2 to 7 from the YTATAWAWA motif has a G in position 7.

In another aspect, the present invention relates to a modified promoter obtained or obtainable by a method according to any of the embodiments described above.

In yet another aspect, the present invention relates to a method of screening a promoter endogenous to a plant for a nine nucleotide motif, which can be modified by introducing one or more point mutations to create a motif having at least one nucleotide and at most three nucleotides, which deviate(s) in positions 2 to 7 from the YTATAWAWA motif in order to increase the expression of the polynucleotide under the control of the promoter comprising the steps:
(i) identifying in the promoter endogenous to a plant, a range of nucleotide positions, in which the introduction of a TATA-box motif of the sequence YTATAWAWA leads to an increased expression of the polynucleotide under the control of the promoter compared to the unmodified promoter;
(ii) screening the range of nucleotide positions identified in step i) to identify a nine nucleotide motif, that can be modified by introducing one or more point mutations to create a motif having at least one nucleotide and at most three nucleotides, which deviate(s) in positions 2 to 7 from the YTATAWAWA motif.

In one embodiment of the method of screening described above, step (i) comprises inserting a YTATAWAWA motif at at least three, preferably at least four, preferably at least five positions and assessing the expression level of the polynucleotide under the control of the promoter for each insertion separately.

### Brief description of sequences

| | |
|---|---|
| SEQ ID NO: 1 | Sequence of corn promoter ZmCWI4 derived from *Zea mays* |
| SEQ ID NO: 2 | Sequence of corn promoter ZmCWI2 derived from *Zea mays* |
| SEQ ID NO: 3 | Sequence of sugar beet promoter BvPAP2 derived from *Beta vulgaris* |
| SEQ ID NO: 4 | Sequence of sugar beet promoter BvEPSPS derived from *Beta vulgaris* |

### Definitions

A "promoter" refers to a DNA sequence capable of controlling and/or regulating expression of a coding sequence, i.e., a gene or part thereof, or of a functional RNA, i.e. a RNA which is active without being translated, for example, a miRNA, a siRNA, an inverted repeat RNA or a hairpin forming RNA. A promoter is usually located at the 5' part of a gene. Promoters can have a broad spectrum of activity, but they can also have tissue or developmental stage specific activity. For example, they can be active in cells of roots, seeds and meristematic cells, etc. A promoter can be active in a constitutive way, or it can be inducible. The induction can be stimulated by a variety of environmental conditions and stimuli. Often promoters are highly regulated. A promoter of the present disclosure may include an endogenous promoter natively present in a cell, or an artificial or transgenic promoter, either from another species, or an artificial or chimeric promoter, i.e. a promoter that does not naturally occur in nature in this composition and is composed of different promoter elements. The promoter of the present invention is preferably an endogenous promoter. The process of transcription begins with the RNA polymerase (RNAP) binding to DNA in the promoter region, which is in the immediate vicinity of the transcription start site (TSS) at the position +1. From analysis in Arabidopsis thaliana the most frequently observed sequence at this position is CA, and TA was the second. There is a strong preference of a dimer sequence at the -1/+1 position. It has been clearly shown that most of the TSS is A or G, and the -1 position is likely to be C or T. This YR Rule (Y: C or T, R: A or G) applies to as many as 77% of the Arabidopsis promoters that is a much higher frequency than expected random appearance (25%) (Yamamoto et al. (2007). Identification of plant promoter constituents by analysis of local distribution of short sequences. BMC genomics, 8(1), 67.). A typical promoter sequence is thought to comprise some regulatory sequence motifs positioned at specific sites relative to the TSS. These cis-regulatory elements are e.g. binding sites for trans-acting factors such as transcription factors. The structure of eukaryotic promoters may be rather complex as they have several different sequence motifs, such as TATA box, INR box, BRE, CCAAT-box and GC-box (Bucher P., J. Mol. Biol. 1990 Apr 20; 212(4):563-78.) or Y Patch promoter elements (Yamamoto et al. (2007). Identification of plant promoter constituents by analysis of local distribution of short sequences. BMC genomics, 8(1), 67; Civáň, P., & Švec, M. (2009). Genome-wide analysis of rice (Oryza sativa L. subsp. japonica) TATA box and Y Patch promoter elements. Genome, 52(3), 294-297.). Promoters can be of varying length and may span more than a thousand nucleotides. Finally, promoter architectures and function differ in different taxa. In particular, there are huge differences between eukaryotic and prokaryotic promoters, but also eukaryotic promoters, e.g., plant promoters and mammalian cell promoters, differ in structure, function and the regulatory network within the cell. Therefore, findings derived from studies with mammalian cell promoters may not necessarily apply for plant promoters within a plant cell environment.

The term "gene expression" or "expression" as used herein refers to the conversion of the information, contained in a gene or nucleic acid molecule, into a "gene product" or "expression product". A "gene product" or "expression product" can be the direct transcriptional product of a gene or nucleic acid molecule (e.g., mRNA, tRNA, rRNA, antisense RNA, ribozyme, structural RNA or any other type of RNA) or a protein produced by translation of an mRNA. Gene products or expression products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

An "increased expression level" in the context of the present invention is determined with respect to the expression level of the unmodified (native) promoter of the genomic locus of interest. For example, expression levels can be determined and compared by quantifying enzyme activity of a reporter gene under the control of the promoter(s). An increased expression level in the context of the present invention is at least 5%, at least 10%, at least 20%, at least 30%, at least 50% with respect to the expression level of the unmodified (native) promoter of the genomic locus of interest. Preferably, the increased expression level in the context of the present invention is at least 2-fold, 5-fold, at least 10-fold, at least 20-fold, at least 30-fold, or at least 40-fold, with respect to the expression level of the unmodified (native) promoter of the genomic locus of interest.

A nucleic acid molecule or polynucleotide that is "endogenous" to a cell or organism refers to a nucleic acid molecule that naturally occurs in the genome of this cell or organism. On the other hand, a nucleic acid molecule that is "exogenous" to a cell or organism refers to a nucleic acid molecule that does not naturally occur in this cell or organism but has been inserted or introduced.

A "TATA box motif' refers to a sequence found in many core promoter regions of eukaryotes. The TATA-box motif is usually found within 100 nucleotides upstream of the transcription start site. It generally contains the consensus sequence 5'-TATA(A/T)A(A/T)-3. In the context of the present invention the sequence YTATAWAWA is used for reference for a TATA box motif, wherein Y stands for C or T and W stands for A or T. A nucleotide, which "deviates" from this sequence in a certain position is therefore in position 2 a G or a C or an A, in position 3, a T or a G or a C, in position 4 a G or a C or an A, in position 5 a T or a G or a C, in position 6 a G or a C and in position 7 a T or a G or a C.

A "point mutation" in the context of the present invention is a single nucleotide exchange, i.e. where one nucleotide is substituted for another one. A point mutation can be introduced e.g. by mutagenesis, wherein "mutagenesis" refers to a technique, by which modifications or mutations are introduced into a nucleic acid sequence in a random or non- site-specific way. For example, mutations can be induced by certain chemicals such as EMS (ethyl methanesulfonate) or ENU (N-ethyl-N-nitrosourea) or physically, e.g., by irradiation with UV or gamma rays. "Site-specific modifications", on the other hand, rely on the action of site-specific effectors such as nucleases, nickases, recombinases, transposases, base editors, prime editors and the like. These tools recognize a certain target sequence and allow to introduce a modification at a specific location within the target sequence.

"TILLING" (Targeting Induced Local Lesions in Genomes) is a process, which allows to identify mutations in a specific gene after an (unspecific) mutagenesis has been performed. Mutagenesis may e.g., be performed using a chemical mutagen such as EMS. Then, a sensitive DNA screening technique is used to identify single base mutations. Methods for performing TILLING are known to the skilled person.

The terms "plant" or "plant cell" as used herein refer to a plant organism, a plant organ, differentiated and undifferentiated plant tissues, plant cells, seeds, and derivatives and progeny thereof. Plant cells include without limitation, for example, cells from seeds, from mature and immature cells or organs, including embryos, meristematic tissues, seedlings, callus tissues in different differentiation states, leaves, flowers, roots, shoots, male or female gametophytes, sporophytes, pollen, pollen tubes and microspores, protoplasts, macroalgae and microalgae. The cells can have any degree of ploidity, i.e. they may either be haploid, diploid, tetraploid, hexaploid or polyploid.

The "start codon of the gene product" refers to the first nucleotide triplet that is translated into an amino acid.

A "modified promoter region" in the context of the present invention refers to a promoter region, which differs in at least one nucleotide from the native or endogenous promoter sequence.

### Detailed description

The present invention provides a novel technique to increase the expression level of a gene of interest in a plant by only introducing minimal modifications. The minimal modifications can be introduced by means such as mutagenesis and do not require the insertion of foreign DNA sequences or the use of genome editing. Thus, it is possible to provide plants with desired traits, which do not qualify as genetically modified organisms. Surprisingly, in the context of the present invention, it was discovered that there are lot more options to create a promoter activating motif than previously believed, which allows a much broader range of application of the methods disclosed herein.

Promoter activating TATA box variants can be used as a tool to increase expression of trait relevant genes by minimal modification of the genomic sequence of crop plants. However, it is challenging to identify all suitable options a target promoter provides to increase its expression by placing just a single point mutation (SNP).

Being able to choose from a large number of functional TATA box variants which also substantially deviate from the TATA box consensus motif is beneficial to identify options in which preferably one nucleotide exchange is sufficient to de novo generate a TATA box motif variant, in a suitable location, in a promoter of interest, or to optimize an already existing TATA box motif. The nucleotide exchange should preferably be a C to T exchange or a G to A (C to T on the reverse strand) as this type of mutation is prevalent in EMS mutagenized populations for TILLING and would provide the possibility to increase expression of target genes in a non-regulated way.

From previous studies, it was known that promoters of many moderately or lowly expressed genes nicely respond to insertion of a perfect consensus TATA box motif (CTATAAATA).

A sequence window of ~100 bp can be determined, in which a target promoter responds to the TATA box insertion. Within this window an optimal position for placing a TATA-box can be identified.

The sequence window, in which a promoter responds to a TATA box is the target region to search for options to generate one of the promoter activating TATA box variants by just one C to T or G to A exchange. The systematic testing of TATA-box variants facilitates the setting up of a search matrix, which identifies options for activating point mutations in the target promoter and at the same time provides an idea if the resulting promoter activation is expected to be low, moderate or strong. Thereby this tool enables a fine-tuned modulation of gene expression.

In a first aspect, the present invention relates to a method for increasing the expression level of an endogenous polynucleotide of interest in a genomic locus of a plant, comprising the steps:
(i) identifying in the promoter region of the genomic locus, a range of nucleotide positions, in which the introduction of a TATA-box motif of the sequence YTATAWAWA leads to an increased expression of the polynucleotide of interest compared to the unmodified genomic locus;
(ii) screening the range of nucleotide positions identified in step i) to identify a nine nucleotide motif, which can be modified by introducing one or more point mutations to create a nine nucleotide motif having at least one nucleotide and at most three nucleotides, which deviate(s) in positions 2 to 7 from a YTATAWAWA motif; and
(iii) introducing, simultaneously and/or subsequently, one or more point mutations, to create a nine nucleotide motif having at least one nucleotide and at most three nucleotides, which deviate(s) in positions 2 to 7 from the YTATAWAWA motif within the range of nucleotide positions identified in step i);
(iv) optionally: obtaining a modified promoter region increasing the expression level of an endogenous polynucleotide of interest in a genomic locus of a plant.

In step (i), the range of nucleotide positions can either be estimated based on experience from analysis of other promoters or, more preferably, is based on transient testing to identify the region responsive to generation of a consensus TATA box. In a preferred embodiment, a TATA box motif of the sequence YTATAWAWA is introduced at several positions within the promoter region. For this purpose, two or three, or preferably four or five positions or more can be chosen and test constructs for each of the selected positions can be produced. The promoter activities of the native (unmodified) promoter and each of the promoter variants with the TATA box insertions in different locations are then compared. For example, the enzymatic activity of a reporter gene driven by the different promoter variants can be used to quantify and compare expression levels. By this method, a range of nucleotide positions can be identified, in which the promoter responds to the presence of a TATA box motif with an increased activity.

In the next step, step (ii), the sequence of the range identified in step (i) is screened for a suitable motif to create a promoter activating TATA box motif by only one, two or a few point mutations. To perform the screening, e.g. sequence analysis software is available to the skilled person. The screening step is explained in more details further below.

The introduction of the one or more point mutations in step (iii) can be performed by various methods known to the skilled person. Examples include the use of site-specific effectors such as nucleases, nickases, recombinases, transposases, base editors, prime editors and the like. Preferably, however, the mutations are introduced by mutagenesis, e.g. by using certain chemicals such as EMS (ethyl methanesulfonate) or ENU (N-ethyl-N-nitrosourea) or physically, e.g., by irradiation with UV or gamma rays.

Preferably, a nine nucleotide motif can be identified in step (ii), which requires only one point mutation to be turned into a promoter activating motif resulting in an increased expression of the gene of interest.

In a preferred embodiment of the method described above, three point mutations, preferably two point mutations, more preferably only one point mutation is/are introduced in step (iii).

In a further preferred embodiment, the point mutation or at least one or at least two point mutation(s) or all point mutations is/are a C to T exchange, preferably introduced by TILLING.

The prevalent mutation in EMS (ethyl methanesulfonate) mutagenesis is a C to T exchange. Therefore, it is preferred that only a single C to T exchange is necessary to create a promoter activating motif resulting in an increased expression of the gene of interest. The C to T mutation can routinely be detected by TILLING as known to the skilled person.

In a preferred embodiment of the method described above, the nine nucleotide motif having at least one nucleotide and at most three nucleotides, which deviate(s) in positions 2 to 7 from the YTATAWAWA motif has a G in position 7.

Surprisingly, a G in position 7 is very well tolerated leading to promoter activating effects comparable or even stronger than the effects of the four possible TATA-box consensus motifs.

Preferably, the nine nucleotide motif having at least one nucleotide and at most three nucleotides, which deviate(s) in positions 2 to 7 from the YTATAWAWA motif and has a G in position 7, is selected from the group consisting of CTATAAGTA, CTATATGTA, CTATAAGAA, CTATATGAA, CCATAAGTA, CTTTAAGTA, CTACAAGTA, CTATTAGTA, CTTTATGTA, CTATTTGTA, CTTTAAGAA, CATTAAGTA, CCTTAAGTA, CGTTAAGTA and CTTCAAGTA.

In another preferred embodiment of the method described above, the range of nucleotide positions identified in step (i) is between minus 400 nucleotides and minus 50 nucleotides upstream of the start codon of the gene product encoded by the polynucleotide of interest.

It has further been established in the context of the present invention that in a range between minus 400 nucleotides and minus 50 nucleotides upstream of the start codon of the gene product, a range of positions can be identified, in which a TATA box insertion results in increased activity of the promoter as evidenced by an increased expression.

In a further preferred embodiment of the method described above, the nine nucleotide motif having at least one nucleotide and at most three nucleotides, which deviate(s) in positions 2 to 7 from the YTATAWAWA motif is selected from the group consisting of CCTTAAATA, CTTTAAATA, CTACTTATA and CTATTTATA.

Advantageously, the method according to the present invention as described in the various embodiments above, is applicable to a wide range of plants and target genes.

In a preferred embodiment of the method described above, the plant is selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus, Beta spp., including Beta vulgaris,* Beta vulgaris subsp. vulgaris *(e.g. Swiss chard, red beet), Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Spinacia oleracea, plants of the family Cucurbitaceae (e,g, Cucumis sativus, Cucumis melo, Citrullus lanatus), plants of the genus Capsicum (e.g. Capsicum annuum), plants of the genus Phaseolus (e.g. P. vulgaris) Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* preferably *Beta vulgaris* and *Zea mays.*

In a further preferred embodiment of the method described above, the polynucleotide of interest is selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance ortolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content.

In a further aspect, the present invention relates to a method of producing a modified promoter region comprising the steps:
(i) identifying in a promoter endogenous to a plant, a range of nucleotide positions, in which the introduction of a TATA-box motif of the sequence YTATAWAWA leads to an increased expression of the polynucleotide under the control of the promoter;
(ii) screening the range of nucleotide positions identified in step i) to identify a nine nucleotide motif, which can be modified by introducing one or more point mutations to create a nine nucleotide motif having at least one nucleotide and at most three nucleotides, which deviate(s) in positions 2 to 7 from a YTATAWAWA motif; and
(iii) introducing, simultaneously and/or subsequently, one or more point mutations, to create a nine nucleotide motif having at least one nucleotide and at most three nucleotides, which deviate(s) in positions 2 to 7 from the YTATAWAWA motif within the range of nucleotide positions identified in step i);
(iv) optionally: obtaining a modified promoter region increasing the expression level of an endogenous polynucleotide of interest in a genomic locus of a plant.

In step (i), the range of nucleotide positions can either be estimated based on experience from analysis of other promoters or, more preferably, is based on transient testing to identify the region responsive to generation of a consensus TATA box. In a preferred embodiment, a TATA box motif of the sequence YTATAWAWA is introduced at several positions within the promoter region. For this purpose, two or three, or preferably four or five positions or more can be chosen and test constructs for each of the selected positions can be produced. The promoter activities of the native (unmodified) promoter and each of the promoter variants with the TATA box insertions in different locations are then compared. For example, the enzymatic activity of a reporter gene driven by the different promoter variants can be used to quantify and compare expression levels. By this method, a range of nucleotide positions can be identified, in which the promoter responds to the presence of a TATA box motif with an increased activity.

In the next step, step (ii), the sequence of the range identified in step (i) is screened for a suitable motif to create a promoter activating TATA box motif by only one, two or a few point mutations. To perform the screening, e.g. sequence analysis software is available to the skilled person.

Publicly available software such as CLC Genomics Workbench by Qiagen can be used in step ii) for convenience. The motif search functionality allows screening with large batches of sequences. However, it is also possible to search for all options one after the other without the use of a "motif search functionality".

As an example:
• TATA-box variant E059a-9-13 CCTTAAATA was identified as suitable for activating a promoter. This variant is having 2 deviations in the inner 6bp (positions 2 to 7) of the consensus TATA-box motif.
• Finding options to generate E059a-9-13, the promoter sequence (the region responding) now needs to be screened for the following sequences which can be converted into E059a-9-13 by a C to T or G to A exchange:

| | |
|---|---|
| >E059a-9-13m1 | CCCTAAATA |
| >E059a-9-13m2 | CCTCAAATA |
| >E059a-9-13m3 | CCTTGAATA |
| >E059a-9-13m4 | CCTTAGATA |
| >E059a-9-13m5 | CCTTAAGTA |
| >E059a-9-13m6 | CCTTAAACA |
| >E059a-9-13m7 | CCTTAAATG |

The introduction of the one or more point mutations in step (iii) can be performed by various methods known to the skilled person. Examples include the use of site-specific effectors such as nucleases, nickases, recombinases, transposases, base editors, prime editors and the like. Preferably, however, the mutations are introduced by mutagenesis, e.g. by using certain chemicals such as EMS (ethyl methanesulfonate) or ENU (N-ethyl-N-nitrosourea) or physically, e.g., by irradiation with UV or gamma rays.

Preferably, a nine nucleotide motif can be identified in step (ii), which requires only one point mutation to be turned into a promoter activating motif resulting in an increased expression of the gene of interest.

In a preferred embodiment of the method described above, three point mutations, preferably two point mutations, more preferably only one point mutation is/are introduced in step (iii).

In a further preferred embodiment, the point mutation or at least one or at least two point mutation(s) or all point mutations is/are a C to T exchange, preferably introduced by TILLING.

The prevalent mutation in EMS (ethyl methanesulfonate) mutagenesis is a C to T exchange. Therefore, it is preferred that only a single C to T exchange is necessary to create a promoter activating motif resulting in an increased expression of the gene of interest. The C to T mutation can routinely be detected by TILLING as known to the skilled person.

In a preferred embodiment of the method described above, the nine nucleotide motif having at least one nucleotide and at most three nucleotides, which deviate(s) in positions 2 to 7 from the YTATAWAWA motif has a G in position 7.

Surprisingly, a G in position 7 is very well tolerated leading to promoter activating effects comparable or even stronger than the effects of the four possible TATA-box consensus motifs.

Preferably, the nine nucleotide motif having at least one nucleotide and at most three nucleotides, which deviate(s) in positions 2 to 7 from the YTATAWAWA motif and has a G in position 7, is selected from the group consisting of CTATAAGTA, CTATATGTA, CTATAAGAA, CTATATGAA, CCATAAGTA, CTTTAAGTA, CTACAAGTA, CTATTAGTA, CTTTATGTA, CTATTTGTA, CTTTAAGAA, CATTAAGTA, CCTTAAGTA, CGTTAAGTA and CTTCAAGTA.

In another preferred embodiment of the method described above, the range of nucleotide positions identified in step (i) is between minus 400 nucleotides and minus 50 nucleotides upstream of the start codon of the gene product encoded by the polynucleotide of interest.

It has further been established in the context of the present invention that in a range between minus 400 nucleotides and minus 50 nucleotides upstream of the start codon of the gene product, a range of position can be identified, in which a TATA box insertion results in increased activity of the promoter as evidenced by an increased expression.

In a further preferred embodiment of the method described above, the nine nucleotide motif having at least one nucleotide and at most three nucleotides, which deviate(s) in positions 2 to 7 from the YTATAWAWA motif is selected from the group consisting of CCTTAAATA, CTTTAAATA, CTACTTATA and CTATTTATA.

In a further aspect, the present invention relates to a modified promoter obtained or obtainable by a method according to any of the embodiments described above.

In another aspect, the present invention relates to a method of screening a promoter endogenous to a plant for a nine nucleotide motif, which can be modified by introducing one or more point mutations to create a motif having at least one nucleotide and at most three nucleotides, which deviate(s) in positions 2 to 7 from the YTATAWAWA motif in order to increase the expression of the polynucleotide under the control of the promoter comprising the steps:
(i) identifying in the promoter endogenous to a plant, a range of nucleotide positions, in which the introduction of a TATA-box motif of the sequence YTATAWAWA leads to an increased expression of the polynucleotide under the control of the promoter compared to the unmodified promoter;
(ii) screening the range of nucleotide positions identified in step i) to identify a nine nucleotide motif, that can be modified by introducing one or more point mutations to create a motif having at least one nucleotide and at most three nucleotides, which deviate(s) in positions 2 to 7 from the YTATAWAWA motif.

In step (i), the range of nucleotide positions can either be estimated based on experience from analysis of other promoters or, more preferably, is based on transient testing to identify the region responsive to generation of a consensus TATA box. In a preferred embodiment, a TATA box motif of the sequence YTATAWAWA is introduced at several positions within the promoter region. For this purpose, two or three, or preferably four or five positions or more can be chosen and test constructs for each of the selected positions can be produced. The promoter activities of the native (unmodified) promoter and each of the promoter variants with the TATA box insertions in different locations are then compared. For example, the enzymatic activity of a reporter gene driven by the different promoter variants can be used to quantify and compare expression levels. By this method, a range of nucleotide positions can be identified, in which the promoter responds to the presence of a TATA box motif with an increased activity.

In a preferred embodiment of the method of screening, step (i) comprises inserting a YTATAWAWA motif at at least three, preferably at least four, preferably at least five positions and assessing the expression level of the polynucleotide under the control of the promoter for each insertion separately.

In the next step, step (ii), the sequence of the range identified in step (i) is screened for a suitable motif to create a promoter activating TATA box motif by only one, two or a few point mutations. To perform the screening, e.g. sequence analysis software is available to the skilled person. The screening step ii) is explained in more detail above.

In a preferred embodiment of the method of screening described above, a nine nucleotide motif can be identified in step (ii), which requires only one point mutation to be turned into a promoter activating motif resulting in an increased expression of the gene of interest.

In a further preferred embodiment of the method of screening described above, the point mutation or at least one or at least two point mutation(s) or all point mutations required to turn the identified motif into a promoter activating motif is/are a C to T exchange.

The prevalent mutation in EMS (ethyl methanesulfonate) mutagenesis is a C to T exchange. Therefore, it is preferred that only a single C to T exchange is necessary to create a promoter activating motif resulting in an increased expression of the gene of interest. The C to T mutation can routinely be detected by TILLING as known to the skilled person.

In a preferred embodiment of the method described above, the nine nucleotide motif having at least one nucleotide and at most three nucleotides, which deviate(s) in positions 2 to 7 from the YTATAWAWA motif has a G in position 7.

Surprisingly, a G in position 7 is very well tolerated leading to promoter activating effects comparable or even stronger than the effects of the four possible TATA-box consensus motifs.

Preferably, the nine nucleotide motif having at least one nucleotide and at most three nucleotides, which deviate(s) in positions 2 to 7 from the YTATAWAWA motif and has a G in position 7, is selected from the group consisting of CTATAAGTA, CTATATGTA, CTATAAGAA, CTATATGAA, CCATAAGTA, CTTTAAGTA, CTACAAGTA, CTATTAGTA, CTTTATGTA, CTATTTGTA, CTTTAAGAA, CATTAAGTA, CCTTAAGTA, CGTTAAGTA and CTTCAAGTA.

In another preferred embodiment of the method described above, the range of nucleotide positions identified in step (i) is between minus 400 nucleotides and minus 50 nucleotides upstream of the start codon of the gene product encoded by the polynucleotide of interest.

It has further been established in the context of the present invention that in a range between minus 400 nucleotides and minus 50 nucleotides upstream of the start codon of the gene product, a range of position can be identified, in which a TATA box insertion results in increased activity of the promoter as evidenced by an increased expression.

In a further preferred embodiment of the method described above, the nine nucleotide motif having at least one nucleotide and at most three nucleotides, which deviate(s) in positions 2 to 7 from the YTATAWAWA motif is selected from the group consisting of CCTTAAATA, CTTTAAATA, CTACTTATA and CTATTTATA.

### Example 1: Transient test system

Bombardment of leaf explants with luciferase reporter constructs. The constructs possess two luciferase reporter genes, one is used for normalization purposes and driven by the 35S promoter, and the other reporter (NLuc) is used for measuring the activity of the target promoter. Each construct is bombarded in 4 replicates, ensuring robustness of the transient test system and statistical evaluation of results.

The readout of the reporter on protein level captures transcriptional as well as translational effects of promoter modifications.

### Example 2: Cloning strategy

The TATA box variants are inserted into the selected promoters at the defined positions by oligo ligation. As a result, the 9 bp TATA box variants are in all tested promoters and positions flanked by the same nucleotides, which are AG in front of the TATA-box and CC downstream of the inserted TATA box variant. This strategy removes a potential bias by differing nucleotides immediately upstream and downstream of the 9 bp TATA-box variants.

**Table 1: Selected promoters and chosen insertion sites for systematic testing of TATA-box variants**

| **Promoter** | **Name of position for TATA-box insertion** | **Position of inserted TATA-box relative to TSS*** | **Position of inserted TATA-box relative to ATG** | **Rating of chosen TATA-box insertion site** | **Rating overall response of the promoter to an inserted TATA-box** | **Window in which the promoter responds to TATA-box insertion (relative to ATG)** |
|---|---|---|---|---|---|---|
| ZmCWI4 SEQ ID NO: 1 | +36 | plus 36 | minus 38 | optimal position | high | minus 144 to minus 17 |
| ZmCWI4 SEQ ID NO: 1 | -60 | minus 60 | minus 126 | non-optimal position | moderate | minus 144 to minus 17 |
| ZmCWI2 SEQ ID NO: 2 | -50 | minus 61 | minus 110 | close to optimal position | moderate | minus 140 to minus 83 |
| BvPAP2 SEQ ID NO: 3 | +107 | plus 107 | minus 195 | optimal position | moderate | minus 248 to minus 103 |
| BvEPSPS SEQ ID NO: 4 | +40 | plus 51 | minus 176 | optimal position | moderate | minus 177 to minus 101 |

| | | | | | | |
|---|---|---|---|---|---|---|
| *TSS (transcription start site) can vary across tissues or developmental stages. We selected the most prominent transcription start site visible in RNAseq data as reference to name the position. Position of inserted TATA-box given with 1st nucleotide of the TATA-box as anchor point. | | | | | | |

### Example 3: nucleotide frequencies matrix for TATA-box motif

Taken from Shahmuradov et al. 2003

**Table 2: Nucleotide frequencies matrix for TATA box from 171 unrelated plant promoters^{a}**

| | < 2 | < 1 | 1 | 2 | 3 | 4 | 5 | 6 | 7 | 8 | >1 | >2 |
|---|---|---|---|---|---|---|---|---|---|---|---|---|
| A | 0.28 | 0.16 | 0.03 | 0.95 | 0.00 | 1.00 | 0.62 | 0.97 | 0.38 | 0.73 | 0.13 | 0.30 |
| C | 0.27 | 0.63 | 0.01 | 0.00 | 0.04 | 0.00 | 0.00 | 0.00 | 0.01 | 0.08 | 0.42 | 0.42 |
| G | 0.17 | 0.05 | 0.00 | 0.00 | 0.00 | 0.00 | 0.00 | 0.02 | 0.00 | 0.10 | 0.28 | 0.16 |
| T | 0.28 | 0.16 | 0.96 | 0.05 | 0.96 | 0.00 | 0.38 | 0.01 | 0.61 | 0.09 | 0.18 | 0.11 |
| | | c | T | A | T | A | A/T | A | T/A | A | | |

| | | | | | | | | | | | | |
|---|---|---|---|---|---|---|---|---|---|---|---|---|
| ^{a}The mean distance between TATA box and TSS is 26 bp. | | | | | | | | | | | | |

The 9 bp TATA-box motif almost exclusively foresees the nucleotide A in position 7 (named as position 6 in Table 2, which was taken from Shahmuradov et al.). Shahmuradov et al., PlantProm- a database of plant promoter sequences, Nucleic Acids Research, 2003, Vol. 31, No. 1, DOI: 10.1093/nar/gkg041

### Example 4: Possible consensus motifs of the TATA-box in plant promoters

Possible consensus motifs of the TATA-box in plant promotors comprise the following sequences:

| | |
|---|---|
| E059a | CTATAAATA |
| E059b | CTATATATA |
| E059c | CTATAAAAA |
| E059d | CTATATAAA |

### Example 5: G in position 7 of the TATA-box motif

The four TATA-box consensus motifs and variants thereof were tested in a transient test system for their capability of activating promoters. Surprisingly, it was found that the nucleotide G in position 7 (Variant ID E059a-21) is so well tolerated that promoter activation is equal or even better compared to insertion of a consensus TATA-box motif.

**Table 3: Promoter activation in transient system compared to unmodified promoter (without inserted TATA-box/TATA-box variant)**

| **variant ID** | **sequence** | **ZmCWI4+36** | **BvPAP2+107** |
|---|---|---|---|
| E059a | CTATAAATA | 63.33 | 14.42 |
| E059b | CTATATATA | 76.90 | 16.93 |
| E059c | CTATAAAAA | 34.77 | 8.68 |
| E059d | CTATATAAA | 39.07 | 10.99 |
| E059a-21 | CTATAAGTA | 74.06 | 19.74 |

### Example 6: TATA-box variants with a G in position 7 have substantial promoter activating effects

**Table 4: Promoter activation in transient system compared to unmodified promoter (without inserted TATA-box/TATA-box variant)**

| **variant ID** | **sequence** | **ZmCWI4+36** | **BvPAP2+107** |
|---|---|---|---|
| E059a-21 | CTATAAGTA | 74.06 | 19.74 |
| E059b-21 | CTATATGTA | 20.50 | 4.21 |
| E059c-21 | CTATAAGAA | 16.27 | 5.63 |
| E059d-21 | CTATATGAA | 3.14 | 2.35 |
| E059a-9-21 | CCATAAGTA | 9.62 | 2.65 |
| E059a-13-21 | CTTTAAGTA | 24.95 | 5.15 |
| E059a-15-21 | CTACAAGTA | 11.66 | 3.57 |
| E059a-19-21 | CTATTAGTA | 11.42 | 4.64 |
| E059b-13-21 | CTTTATGTA | 2.07 | 1.87 |
| E059b-19-21 | CTATTTGTA | 4.15 | 2.48 |
| E059c-13-21 | CTTTAAGAA | 2.61 | 2.01 |
| E059a-13-21-8 | CATTAAGTA | 4.06 | 2.69 |
| E059a-13-21-9 | CCTTAAGTA | 5.55 | 3.21 |
| E059a-13-21-10 | CGTTAAGTA | 4.40 | 2.54 |

### Example 7: C to T exchange leading to promoter activation by generating TATA-box variant E059a-21

**Table 5: The screening step (ii) identifies the following SEQ:**

| **variant ID** | **sequence** | **ZmCWI4+36** | **BvPAP2+107** |
|---|---|---|---|
| E059a-9-21 | CCATAAGTA | 9.62 | 2.65 |

**Table 6: Modification in step (iii) changes C to T in position 2, which increases the promoter activity considerably**

| **variant ID** | **sequence** | **ZmCWI4+36** | **BvPAP2+107** |
|---|---|---|---|
| E059a-21 | CTATAAGTA | 74.06 | 19.74 |

### Example 8: C to T exchange leading to promoter activation by generating TATA-box variant E059a-13-21

**Table 7: The screening step (ii) identifies the following SEQ:**

| **variant ID** | **sequence** | **ZmCWI4+36** | **BvPAP2+107** |
|---|---|---|---|
| E059a-13-21-15 | CTTCAAGTA | 1.87 | 1.26 |

**Table 8: Modification in step (iii) changes a C to T in position 4, which increases the promoter activity considerably**

| **variant ID** | **sequence** | **ZmCWI4+36** | **BvPAP2+107** |
|---|---|---|---|
| E059a-13-21 | CTTTAAGTA | 13.57 | 2.61 |

### Example 9: C to T exchange leading to promoter activation by generating TATA-box variant E059a-13

**Table 9: The screening step (ii) identifies the following SEQ:**

| **variant ID** | **sequence** | **ZmCWI4+36** | **BvPAP2+107** |
|---|---|---|---|
| E059a-9-13 | CCTTAAATA | 28.63 | 6.24 |

**Table 10: Modification in step (iii) changes a C to T in position 2, which increases the promoter activity considerably**

| **variant ID** | **sequence** | **ZmCWI4+36** | **BvPAP2+107** |
|---|---|---|---|
| E059a-13 | CTTTAAATA | 35.61 | 12.91 |

### Example 10: C to T exchange leading to promoter activation by generating TATA-box variant E059b-19

**Table 11: The screening step (ii) identifies the following SEQ:**

| **variant ID** | **sequence** | **ZmCWI4+36** | **BvPAP2+107** |
|---|---|---|---|
| E059b-15-19 | CTACTTATA | 11.01 | 6.12 |

**Table 12: Modification in step (iii) changes a C to a T in position 4, which increases the promoter activity considerably**

| **variant ID** | **sequence** | **ZmCWI4+36** | **BvPAP2+107** |
|---|---|---|---|
| E059b-19 | CTATTTATA | 70.21 | 12.16 |

## Claims

1. A method for increasing the expression level of an endogenous polynucleotide of interest in a genomic locus of a plant, comprising the steps:
(i) identifying in the promoter region of the genomic locus, a range of nucleotide positions, in which the introduction of a TATA-box motif of the sequence YTATAWAWA leads to an increased expression of the polynucleotide of interest compared to the unmodified genomic locus;
(ii) screening the range of nucleotide positions identified in step i) to identify a nine nucleotide motif, which can be modified by introducing one or more point mutations to create a nine nucleotide motif having at least one nucleotide and at most three nucleotides, which deviate(s) in positions 2 to 7 from a YTATAWAWA motif; and
(iii) introducing, simultaneously and/or subsequently, one or more point mutations, to create a nine nucleotide motif having at least one nucleotide and at most three nucleotides, which deviate(s) in positions 2 to 7 from the YTATAWAWA motif within the range of nucleotide positions identified in step i);
(iv) optionally: obtaining a modified promoter region increasing the expression level of an endogenous polynucleotide of interest in a genomic locus of a plant.

2. The method according to claim 1, wherein three point mutations, preferably two point mutations, more preferably only one point mutation is/are introduced in step (iii).

3. The method according to claim 1 or 2, wherein the point mutation or at least one or at least two point mutation(s) or all point mutations is/are a C to T exchange, preferably introduced by TILLING.

4. The method according to any of the preceding claims, wherein the nine nucleotide motif having at least one nucleotide and at most three nucleotides, which deviate(s) in positions 2 to 7 from the YTATAWAWA motif has a G in position 7.

5. The method according to any of the preceding claims, wherein the range of nucleotide positions identified in step (i) is between minus 400 nucleotides and minus 50 nucleotides upstream of the start codon of the gene product encoded by the polynucleotide of interest.

6. The method according to any of claims 1 to 3 or 5, wherein the nine nucleotide motif having at least one nucleotide and at most three nucleotides, which deviate(s) in positions 2 to 7 from the YTATAWAWA motif is selected from the group consisting of CTATAAGTA, CTATATGTA, CTATAAGAA, CTATATGAA, CCATAAGTA, CTTTAAGTA, CTACAAGTA, CTATTAGTA, CTTTATGTA, CTATTTGTA, CTTTAAGAA, CATTAAGTA, CCTTAAGTA, CGTTAAGTA, CTTCAAGTA, CCTTAAATA, CTTTAAATA, CTACTTATA and CTATTTATA.

7. The method according to any of the preceding claims, wherein the plant is selected from the group consisting of
*Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale, Triticale, Malus domestica, Brachypodium distachyon, Hordeum marinum, Aegilops tauschii, Daucus glochidiatus, Beta spp., including Beta vulgaris,* Beta vulgaris subsp. vulgaris *(e.g. Swiss chard, red beet), Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Spinacia oleracea, plants of the family Cucurbitaceae (e,g, Cucumis sativus, Cucumis melo, Citrullus lanatus), plants of the genus Capsicum (e.g. Capsicum annuum), plants of the genus Phaseolus (e.g. P. vulgaris) Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and *Allium tuberosum,* preferably *Beta vulgaris* and *Zea mays.*

8. The method according to any of the preceding claims, wherein the polynucleotide of interest is selected from a nucleic acid molecule encoding resistance or tolerance to abiotic stress, including drought stress, osmotic stress, heat stress, cold stress, oxidative stress, heavy metal stress, nitrogen deficiency, phosphate deficiency, salt stress or waterlogging, herbicide resistance, including resistance to glyphosate, glufosinate/phosphinotricin, hygromycin, resistance ortolerance to 2,4-D, protoporphyrinogen oxidase (PPO) inhibitors, ALS inhibitors, and Dicamba, a nucleic acid molecule encoding resistance or tolerance to biotic stress, including a viral resistance gene, a fungal resistance gene, a bacterial resistance gene, an insect resistance gene, or a nucleic acid molecule encoding a yield related trait, including lodging resistance, flowering time, shattering resistance, seed color, endosperm composition, or nutritional content.

9. A method of producing a modified promoter region comprising the steps:
(i) identifying in a promoter endogenous to a plant, a range of nucleotide positions, in which the introduction of a TATA-box motif of the sequence YTATAWAWA leads to an increased expression of the polynucleotide under the control of the promoter;
(ii) screening the range of nucleotide positions identified in step i) to identify a nine nucleotide motif, which can be modified by introducing one or more point mutations to create a nine nucleotide motif having at least one nucleotide and at most three nucleotides, which deviate(s) in positions 2 to 7 from a YTATAWAWA motif; and
(iii) introducing, simultaneously and/or subsequently, one or more point mutations, to create a nine nucleotide motif having at least one nucleotide and at most three nucleotides, which deviate(s) in positions 2 to 7 from the YTATAWAWA motif within the range of nucleotide positions identified in step i);
(iv) optionally: obtaining a modified promoter region increasing the expression level of an endogenous polynucleotide of interest in a genomic locus of a plant.

10. The method according to claim 9, wherein three point mutations, preferably two point mutations, more preferably only one point mutation is/are introduced in step (iii).

11. The method according to claim 9 or 10, wherein the point mutation or at least one or at least two point mutation(s) or all point mutations is/are a C to T exchange, preferably introduced by TILLING.

12. The method according to any of claims 9 to 11, wherein the nine nucleotide motif having at least one nucleotide and at most three nucleotides, which deviate(s) in positions 2 to 7 from the YTATAWAWA motif has a G in position 7.

13. The modified promoter obtained or obtainable by a method according to any of claims 9 to 12.

14. A method of screening a promoter endogenous to a plant for a nine nucleotide motif, which can be modified by introducing one or more point mutations to create a motif having at least one nucleotide and at most three nucleotides, which deviate(s) in positions 2 to 7 from the YTATAWAWA in order to increase the expression of the polynucleotide under the control of the promoter comprising the steps:
(i) identifying in the promoter endogenous to a plant, a range of nucleotide positions, in which the introduction of a TATA-box motif of the sequence YTATAWAWA leads to an increased expression of the polynucleotide under the control of the promoter compared to the unmodified promoter;
(ii) screening the range of nucleotide positions identified in step i) to identify a nine nucleotide motif, that can be modified by introducing one or more point mutations to create a motif having at least one nucleotide and at most three nucleotides, which deviate(s) in positions 2 to 7 from the YTATAWAWA motif.

15. The method according to claim 14, wherein step (i) comprises inserting a YTATAWAWA motif at at least three, preferably at least four, preferably at least five positions and assessing the expression level of the polynucleotide under the control of the promoter for each insertion separately.
